Europäisches Patentamt

European Patent Office

Office européen des brevets

(11) Veröffentlichungsnummer: **0 592 475 B1**

(12) **EUROPÄISCHE PATENTSCHRIFT**

(45) Veröffentlichungstag der Patentschrift: **13.12.95**

(51) Int. Cl.6: **C11D 1/83**, C11D 1/831, A61K 7/08

(21) Anmeldenummer: **92912550.8**

(22) Anmeldetag: **22.06.92**

(86) Internationale Anmeldenummer:
**PCT/EP92/01399**

(87) Internationale Veröffentlichungsnummer:
**WO 93/00417 (07.01.93 93/02)**

Die Akte enthält technische Angaben, die nach dem Eingang der Anmeldung eingereicht wurden und die nicht in dieser Patentschrift enthalten sind.

(54) **WÄSSRIGE TENSIDZUBEREITUNGEN**

(30) Priorität: **29.06.91 DE 4121612**

(43) Veröffentlichungstag der Anmeldung:
**20.04.94 Patentblatt 94/16**

(45) Bekanntmachung des Hinweises auf die Patenterteilung:
**13.12.95 Patentblatt 95/50**

(84) Benannte Vertragsstaaten:
**AT BE DE ES FR NL**

(56) Entgegenhaltungen:
EP-A- 0 105 556
EP-A- 0 384 983
WO-A-86/02943

(73) Patentinhaber: **Henkel Kommanditgesellschaft auf Aktien**

**D-40191 Düsseldorf (DE)**

(72) Erfinder: **MÜLLER, Reinhard**
**Gottfried-Hausmann-Weg 13**
**D-5140 Erkelenz 7 (DE)**
Erfinder: **BERNICKE, Jutta**
**Händelstrasse 26**
**D-4010 Hilden (DE)**
Erfinder: **SEIDEL, Kurt**
**Nosthoffenstrasse 59**
**D-4000 Düsseldorf 13 (DE)**

EP 0 592 475 B1

**Beschreibung**

Gegenstand der Erfindung sind wäßrige Zubereitungen oberflächenaktiver Stoffe, die eine erhöhte Viskosität aufweisen, und die eine ternäre Kombination von Alkylglykosiden, anorganischen Elektrolytsalzen und speziellen Polyetherverbindungen enthalten.

Es ist bekannt, daß sich die Viskosität wäßriger Tensidlösungen durch Zugabe anorganischer Elektrolytsalze anheben läßt. Diese Eigenschaft von Tensiden erhöht ihren anwendungstechnischen Wert zur Herstellung flüssiger Reinigungsmittel, insbesondere flüssiger Körperreinigungsmittel, flüssiger Seifen und Haarshampoos beträchtlich, da diese Produkte auch bei Anwendungskonzentrationen noch eine merkliche Viskosität aufweisen sollen, damit sie sich leichter von Hand auf dem Kopf oder dem Körper verteilen lassen.

Es sind auch wäßrige Tensidsysteme bekannt, bei denen die Viskosität durch Verdickungsmittel mit hohem Molekulargewicht, z.B. Zuckerester, Polyolestern oder Polyolalkylethern, angehoben wird, vergl. z.B. H. Meijer, Seifen-Öle-Fette-Wachse, 1987 (113) 135.

Aus WO 86/2943 ist bekannt, daß sich die Viskosität von Flüssigwaschmitteln mit Alkylmonoglycosiden mit einem mittleren Oligomerisationsgrad von weniger als 1,4 anheben läßt.

Nach der deutschen Patentanmeldung DE 39 05 939 (EP A 0 384 983) lassen sich wäßrige Tensidkombinationen von Alkyloligoglycosiden und carboxymethylierten Oxethylaten mit Elektrolyten verdikken.

Es besteht jedoch ein ständiger Bedarf an wäßrigen Zubereitungen oberflächenaktiver Substanzen, die über eine verbesserte Verdickbarkeit verfügen und sich dadurch ganz besonders für den Einsatz in Körperreinigungsmittel eignen.

Es wurde nun überraschend gefunden, daß sich wäßrige Systeme von anionischen Tensiden durch geringe Mengen einer ternären Kombination aus Alkylglycosiden, anorganischen Elektrolytsalzen sowie speziellen Polyetherverbindungen außerordentlich gut verdicken lassen.

Gegenstand der Erfindung sind folglich wäßrige Zubereitungen oberflächenaktiver Substanzen enthaltend

(A) 1 bis 30 Gew.-% eines oder mehrerer Aniontenside, die 1 oder 2 lipophile Reste mit 1 bis 22 C-Atomen und 1 bis 3 polare Reste ausgewählt aus der Gruppe der Carboxylat-, Sulfat- oder Sulfonatreste und gegebenenfalls einen Polyoxyalkylenrest mit einem mittleren Alkoxylierungsgrad von 1 bis 15 enthalten,

(B) ein oder mehrere Alkylglycoside der allgemeinen Formel (I), worin

$$RO(G)_x \qquad (I)$$

R einen linearen, gesättigten Alkylrest mit 8 bis 22 C-Atomen und $(G)_x$ ein Glycosid- oder Oligoglycosidrest mit einem Oligomerisationsgrad x von 1 bis 4 ist,

(C) ein anorganisches Elektrolytsalz,

(D) ein oder mehrere Polyetherverbindungen, die 2 oder 3 lipophile Reste mit 8 bis 22 C-Atomen und 1 bis 3 Polyoxyethylenreste mit 20 bis 250 Oxyalkylen-Einheiten enthalten,

wobei die Summe der Komponenten (B), (C) und (D) 0,5 bis 20 Gew.-% beträgt und nicht größer als der Gehalt an Komponente (A) ist.

Unter Polyoxyalkylenrest wird dabei eine Gruppe verstanden, die aus Oxyethylen-Einheiten $-[CH_2-CH_2-O]-$ oder aus Oxypropylen-Einheiten $-[CH(CH_3)-CH_2-O]-$ aufgebaut ist. Die mittlere Anzahl der Oxyethylen- bzw. Oxypropylen-Einheiten wird dabei als mittlerer Alkoxylierungsgrad bezeichnet.

Besonders geeignet sind solche wäßrigen Zubereitungen gemäß der Erfindung, worin der Anteil der oberflächenaktiven Substanz (A) 5 bis 25 Gew.-% beträgt. Die Summe der Komponenten (B), (C) und (D) beträgt vorzugsweise 1,0 bis 10,0 Gew.-%.

Die günstigsten Effekte werden erhalten, wenn die erfindungsgemäßen wäßrigen Zusammensetzungen Alkylglycosid (B) und Elektrolytsalz (C) im Gewichtsverhältnis 5 : 1 bis 1 : 5, vorzugsweise 2 : 1 bis 1 : 3, und Alkylglycosid (B) und Polyetherverbindung (D) im Gewichtsverhältnis 5 : 1 bis 1 : 3, vorzugsweise 2 : 1 bis 1 : 2, enthalten.

Die **Aniontenside (A)** werden erfindungsgemäß bevorzugt ausgewählt aus der Gruppe der Alkyl- und Dialkylethersulfate, Ethercarbonsäuren, Sulfosuccinate, Hydroxyalkylethersulfonate, Fettalkoholsulfate, α-Olefinsulfonate, sekundären Alkylsulfonate, Acylsarkoside, Acyltauride, Fettalkoholethertartrate, Fettalkoholethercitrate und der Sulfierungsprodukte ungesättigter Fettsäuren. Die Cegenionen der Carboxylat-, Sulfat- oder Sulfonatreste werden bevorzugt aus der Gruppe der Alkali- und Erdalkalimetalle, Aluminium, Ammonium sowie Alkyl- oder Alkylolammoniumgruppen mit 1 bis 4 C-Atomen in jeder Alkyl- oder Alkylolgruppe gewählt.

Die chemischen Strukturen sowie die grundlegenden Tensideigenschaften der meisten dieser Aniontenside gehören mittlerweile zum Lehrbuchwissen und bedürfen daher keiner weiteren Erläuterung. Unter **Dialkylethersulfaten** sind Verbindungen zu verstehen wie sie in der Europäischen Patentameldung EP 299 370 beschrieben sind. Aus dieser Schrift können Einzelheiten des Herstellungsverfahrens und der Eigenschaften dieser Verbindungen entnommen werden. Unter **Fettalkoholethertartraten** sind Monoestersalze der Weinsäure, unter **Fettalkoholethercitraten** Mono-und/oder Diestersalze der Zitronensäure mit Anlagerungsprodukten von Ethylenoxid und/oder Propylenoxid an Fettalkohole zu verstehen. **Hydroxyalkylethersulfonate** sind Gegenstand der Deutschen Patentameldung DE 37 25 030. Einzelheiten des Herstellungsverfahrens können der genannten Patentanmeldung DE 37 25 030 entnommen werden. Unter **Sulfonaten von ungesättigten Fettsäuren** sind Sulfonierungsprodukte von Fettsäuren mit 12 bis 22 C-Atomen und 1 bis 6 Doppelbindungen zu verstehen. Derartige Produkte sind literaturbekannt und beispielsweise durch Umsetzung dieser Fettsäuren mit gasförmigem Schwefeltrioxid zugänglich. Am Beispiel der Ölsäure können Einzelheiten des Herstellungsverfahrens der deutschen Patentanmeldung DE 39 26 344 entnommen werden.

Ganz besonders geeignete Aniontenside (A) sind Alkyl- und Dialkylethersulfate sowie Sulfobernsteinsäurehalbester, Fettalkoholethertartrate und Fettalkoholethercitrate.

**Alkylglycoside (B)** der allgemeinen Formel (I) sind seit langem bekannte oberflächenaktive Stoffe, die aus Zuckern und aliphatischen, primären Alkoholen mit 8 - 22 C-Atomen unter Acetalisierung herstellbar sind. Als Zuckerkomponenten (Glycosen) kommen bevorzugt Glucose, daneben aber auch Fructose, Mannose, Galactose, Telose, Gulose, Allose, Altrose, Idose, Arabinose, Xylose, Lyxose, Libose und Gemische davon in Frage.

$$R\text{-}(G)_x \qquad (I)$$

Bevorzugt wegen der leichten Zugänglichkeit und der guten Anwendungseigenschaften sind die Acetalisierungsprodukte der Glucose mit Fettalkoholen R-OH, die z. B. aus natürlichen Fetten und Ölen nach bekannten Verfahren erhältlich sind, insbesondere mit linearen, primären, gesättigten und ungesättigten Fettalkoholen mit 8 bis 22 C-Atomen.

Alkylglycoside der Formel (I), ihre Herstellung und Verwendung als oberflächenaktive Stoffe sind beispielsweise aus US-A-3.839.318, US-A-3.707.535, US-A-3.547.828, DE-A-1943689, DE-A-2036472, DE-A-3001064 sowie EP-A-77167 bekannt. Bezüglich des Glycosidrestes - $(G)_x$ gilt, daß sowohl Monoglycoside ($x$ = 1), bei denen ein Zuckerrest glycosidisch mit dem Fettalkohol verbunden ist, als auch oligomere Glycoside mit einem Oligomerisationsgrad $x$ = 2 bis 4 geeignet sind. In der Regel liegen Gemische von Mono- und Oligoglycosiden vor.

Bevorzugt geeignet sind Alkylglycoside (B) der Formel (I), in welchen R eine Alkylgruppe mit 8 bis 22 C-Atomen und $(G)_x$ ein Glycosid- oder Oligoglycosidrest mit einem Oligomerisationsgrad $x$ = 1 bis 4 ist. Ganz besonders bevorzugt ist R eine Alkylgruppe mit 10 bis 16 C-Atomen, und $(G)_x$, der Rest eines Gemisches von Glucosid und Oligucosiden mit einem mittleren Oligomerisationsgrad von 1 bis 1,5.

Bei Aniontensiden, die einen Polyoxyalkylenrest enthalten, gilt bezüglich des Alkoxylierungsgrades ganz allgemein, daß bei Alkoxylierungsreaktionen wie beispielsweise der Anlagerung von x mol Ethylenoxid an 1 mol Fettalkohol nach den bekannten Ethoxylierungsverfahren kein einheitliches Addukt, sondern ein Gemisch aus Restmengen freien Fettalkohols und einer Reihe homologer (oligomerer) Anlagerungsprodukte von 1, 2, 3, ... x, x+1, x+2 ...usw. Molekülen Ethylenoxid je Molekül Fettalkohol erhalten wird. Der mittlere Ethoxylierungsgrad (x) wird dabei definiert durch die Ausgangsmengen an Fettalkohol und Ethylenoxid. Die Verteilungskurve des Homologengemisches weist in der Regel ein Maximum im Bereich zwischen x-3 und x+3 auf. Nähere Informationen hierzu können beispielsweise der Zeitschrift Soap/Cosmetics/Chemical Specialities, Heft Januar 1988, S. 34, entnommen werden. Neben den üblichen aus dem Stand der Technik bekannten Alkoxylierungskatalysatoren wie Natriummethanolat lassen sich dabei aber auch solche Katalysatoren verwenden, die zu Produkten mit sogenannter eingeengter Homologenverteilung führen, vergl. z.B. Seifen-Öle-Fette-Wachse 1990 (116) 60.

Als **anorganische Elektrolytsalze (C)** eignen sich alle wasserlöslichen Alkali-, Ammonium- und Erdalkalisalze, z. B. die Fluoride, Chloride, Bromide, Sulfate, Phosphate und Nitrate und Hydrogencarbonate, soweit sie in einer Menge von wenigstens 1 Gew.-% bei 20°C in Wasser löslich sind. Bevorzugt werden die Chloride oder Sulfate eines Alkalimetalls, des Ammoniums oder des Magnesiums verwendet; besonders bevorzugt sind Natriumchlorid und Magnesiumchlorid.

Unter **Polyetherverbindungen (D)** im Sinne der Erfindung sind Derivate von Polyolverbindungen mit 2 bis 6 OH-Gruppen zu verstehen, bei denen 2 bis 3 OH-Gruppen über Polyoxyalkylenreste mit insgesamt 20 bis 250 EO-Einheiten mit lipophilen Alkylresten mit jeweils 8 bis 22 C-Atomen verbunden sind. Derartige Produkte sind zum Teil kommerziell verfügbar, z.B. Talgfettalkohol-60EO-myristylglykol, Polyethylenglykol-120-methylglucosid-dioleat oder Propylenglykol-55 EO-dioleat.

Die erfindungsgemäßen Mittel können in einer Vielzahl von reinigenden Konsumentenprodukten wie Haarshampoos, Schaumbädern, Duschbädern, flüssigen Seifen und manuellen Geschirrspülmitteln Verwendung finden.

Diese Produkte enthalten neben Tensiden oder Tensidkombinationen üblicherweise Bestandteile wie Emulgatoren, Ölkomponenten, Lösungsvermittler, Verdickungsmittel, Überfettungsmittel, biogene Wirkstoffe, Filmbildner, Duftstoffe, Farbstoffe, Perlglanzmittel, Schaumstabilisatoren, Konservierungsmittel und pH-Regulatoren. Die erfindungsgemäßen Mittel können daher zusätzliche Komponenten und Hilfsstoffe enthalten, wie sie aus dem Stand der Technik bekannt sind. Die wichtigsten sind:

**Tenside/Emulgatoren**, z.B. anionaktive Tenside mit Carboxylat-, Sulfonat-, Sulfat- oder Phosphatgruppen wie Seifen, Alkyl- und Arylethersulfate, Fettamine, quartäre Ammonium- und Pyridiniumverbindungen, nichtionische Emulgatoren wie Ethylenoxidaddukte an Alkohole, Carbonsäuren, Partialglyceride und Sorbitanester, amphotere und zwitterionische Emulgatoren wie Imidazolinderivate, Betaine oder Sulfobetaine sowie z.B. Fettsäureester und Sorbitanfettsäureester (vergl. z.B. W. Umbach [Hrsg.], "Kosmetik - Entwicklung, Herstellung und Anwendung kosmetischer Mittel", S.86-87, Stuttgart 1988).

**Ölkomponenten**, z.B. Substanzen wie Paraffinöl, Pflanzenöle, Fettsäureester, Squalan und 2-Octyldodecanol; als Fette und Wachse beispielsweise Walrat, Bienenwachs, Montanwachs, Paraffin und Cetylstearylalkohol.

**Lösungsvermittler**, z.B. niedrige ein- oder mehrwertige Alkohole wie Ethanol, Isopropanol, Ethylenglykol, Propylenglykol, Glycerin, 1,3-Butylenglykol und Diethylenglykol.

**Verdickungsmittel**, z.B. Polysaccharide, insbesondere Xanthan-Gum, Guar-Gum, Agar-Agar, Alginate und Tylosen, sowie Carboxymethylcellulose und Hydroxyethylcellulose, ferner höhermolekulare Polyethylenglykolmono- und -diester von Fettsäuren, Polyacrylate, Polyvinylalkohol und Polyvinylpyrrolidon.

**Überfettungsmittel**, z.B. polyethoxylierte Lanolinderivate, Lecithinderivate und Fettsäurealkanolamide, wobei die letzteren gleichzeitig auch als Schaumstabilisatoren dienen.

**Biogenen Wirkstoffe** wie Pflanzenextrakte, Eiweißabbauprodukte und Vitaminkomplexe.

**Filmbildner** wie Polyvinylpyrrolidon, Vinylpyrrolidon-Vinylacetat-Copolymerisate, Polymere der Acrylsäurereihe, quaternäre Cellulose-Derivate und ähnliche Verbindungen.

**Feuchthaltemittel**, z.B. Glycerin, Polyglycerine, Sorbit, 1,2-Propandiol, 1,2,3-Butan-triol, Polyethylenglykole, Glucose, Mannit, Xylit, Pyrollidon-Carbonsäure-Salze (PCA), Aminosäuren, Milchsäure.

**Antimikrobiell wirksame Stoffe** als Konservierungsmittel, z.B. Benzoesäure, Salicylsäure, Sorbinsäure, sowie deren Ester und Salze sowie die in der Anlage zur Kosmetikverordnung aufgeführten Substanzen.

**Perlglanzmittel** wie Glykoldistearinsäureester, Ethylenglykoldistearat oder Fettsäuremonoglykolester.

**Duftstoffe**, z.B. natürliche Riechstoffe, die durch Destillation, Extraktion oder Pressung aus Pflanzen gewonnen werden sowie synthetisch hergestellte Riechstoffe (vergl. z.B. H.Aebi, E.Baumgartner, H.P.Fiedler, G.Ohloff, "Kosmetika, Riechstoffe und Lebensmittelzusatzstoffe", Stuttgart 1978).

**Antioxidantien**, z.B. Tocopherole, Lecithin, Guajakol, Butylkresol, 4-Methyl-2,6-di-tert.-butyl-phenol (BHT), 4-Methoxy-2(3)tert.-butylphenol (BHA)

Farbstoffe, wie sie z.B. von der Farbstoff-Kommission der Deutschen Forschungsgemeinschaft für Kosmetika zusammengestellt sind ("Färbemittel für Kosmetika" Mitteilung 3, Wiesbaden 1968). Die Farbstoffe werden üblicherweise in Konzentrationen von 0,001 bis 0,1 Gew.-%, bezogen auf die gesamte Mischung, eingesetzt.

Weitere Komponenten der erfindungsgemäßen Mittel sind gewünschtenfalls Substanzen, die der Einstellung des pH-Wertes der Mittel dienen.

Die Gesamtmenge der Hilfsstoffe beträgt 0 - 20 Gew.-%, vorzugsweise 0 - 10 Gew.-%.

Zur Herstellung der erfindungsgemäßen wäßrigen Zubereitungen werden die Alkylglycoside (B) und die Polyolverbindungen (D) bei 60 bis 80 °C zu einer wäßrigen Phase gegeben, die die Aniontenside (A) und das Elektrolytsalz (C) enthält. Diese Mischungen werden gerührt und dann auf Normaltemperatur (20 bis 40 °C) abgekühlt. Wegen der Wasserlöslichkeit der Alkylglycoside, insbesondere solcher mit Alkylresten von 12 bis 16 C-Atomen, können die erfindungsgemäßen Zubereitungen vorteilhaft auch auf kaltem Wege hergestellt werden. Dabei werden die Komponenten in einfacher Weise bei Normaltemperatur zusammengerührt.

Darüber hinaus hat es sich als vorteilhaft erwiesen, zunächst Konzentrate der einzelnen Komponenten herzustellen und diese dann - gegebenenfalls nach weiterer Lagerung oder Transport - mit Wasser zu den erfindungsgemäßen Zubereitungen zu verdünnen. Diese Arbeitsweise bietet den Vorteil der Arbeitsökonomie und/oder der Minimierung eventueller Transportkosten.

Die nachfolgenden Beispiele dienen der Erläuterung der Erfindung und sind nicht einschränkend aufzufassen.

**Beispiele**

## 1. Allgemeines

### 1.1. Abkürzungen

(a) Manche Hersteller charakterisieren den Ethoxylierungsgrad von Tensiden durch das Kürzel PEG ("Polyethylenglykol") in Kombination mit einer nachgestellten Zahl; diese Kennzeichnung wird hier bei den Polyetherverbindungen (D) beibehalten.
(b) Zur Kennzeichnung des Gehalts wäßriger Tensidlösungen an Aktivsubstanz wird die Abkürzung **AS-Gehalt** verwendet.
(c) In den Kopfzeilen der Tabellen 1 bis 4 sind die Beispiele mit **B1**, **B2**, **B3**, usw., die Vergleiche mit **V1**, **V2**, **V3** usw. kenntlich gemacht.

### 1.2. Verwendete Substanzen

#### 1.2.1. Tenside (A)

**Texapon N25**: Wäßrige Lösung von Natriumlaurylethersulfat; AS-Gehalt: 28 Gew.-% ("Texapon$^{(R)}$ N25"; Fa. Henkel/Düsseldorf)
**Texapon SB3**: Wäßrige Lösung von Sulfobernsteinsäure $C_{12/14}$-EO-halbester, Na-Salz. AS-Gehalt: 40 Gew.-% ("Texapon$^{(R)}$ SB3"; Fa. Henkel/Düsseldorf
**Texapon ASV**: Wäßrige Lösung spezieller Alkyl- und Alkylethersulfate, CTFA-Bezeichnung: sodium laureth sulfate and magnesium laureth sulfate and sodium laureth-8-sulfate and magnesium laureth-8-sulfate and sodium oleate sulfate and magnesium oleate sulfate. AS-Gehalt: 28 Gew.-% ("Texapon$^{(R)}$ ASV"; Fa. Henkel/Düsseldorf)
**ÖDS**: Wäßrige Lösung von Ölsäuresulfonat,Di-Natriumsalz, hergestellt durch Sulfonierung von technischer Ölsäure mit gasförmigem Schwefeltrioxid gemäß der Deutschen Patentanmeldung DE 39 26 344; dabei wurde die auf Seite 4 dieser Anmeldung näher gekennzeichnete Ölsäure Nr.1 (ex Rindertalg) verwendet. AS-Gehalt: 51 Gew.-%
**$C_{12/14}$-Disalz**: Wäßrige Paste von $\alpha$-Sulfo-$C_{12/14}$-Fettsäure-Di-Natriumsalz, hergestellt durch Sulfonierung eines Gemisches von Laurin- und Myristinsäure mit gasförmigem Schwefeltrioxid. AS-Gehalt: 25 Gew.-%.
**CT-12**: Kokosacylglutamat, Triethanolammoniumsalz (Fa. Ajinomoto/Japan)
**HMS**: Wäßrige Lösung eines Dialkylethersulfats der Formel (III), in der $R^2$ einen n-Decylrest, $R^3$ einen n-Octylrest, $R^4$ Wasserstoff, m die Zahl 2 und b die Zahl 4 bedeuten. AS-Gehalt: 40 Gew.-%

#### 1.2.2. Alkylglycoside (B)

**APG600**: $C_{12/14}$-Fettalkoholglucosid mit einem Oligomerisationsgrad von 1,45 (Fa. Henkel/Düsseldorf).

#### 1.2.3. Polyetherverbindungen (D)

**GT-282**: Talgfettalkohol-60 EO-myristyl-glykol (Fa. Akzo)
**DOE-120**: PEG-120-methylglucosid-dioleat (Fa. Amerchol)
**Antil-141**: Propylenglycol-PEG-55-dioleat (Fa. Goldschmidt/Essen)

## 2. Herstellung der wäßrigen Tensidzubereitungen

Die Zubereitungen der Beispiele B1 bis B10 sowie der Vergleichsbeispiele V1 bis V28 wurden durch Vermischen der Komponenten bei 20 °C hergestellt. Die Viskosität wurde mit Hilfe eines Brookfield-Rotationsviskosimeters (Spindel 3) bestimmt.
Die Ergebnisse der Messungen sind in den Tabellen 1 bis 4 zusammengestellt. Es wird deutlich, daß bei Verwendung der erfindungsgemäßen Zubereitungen, d.h. in Anwesenheit der ternären Kombinationen aus Alkylglucosiden (A), anorganischen Elektrolytsalzen (C) und den Polyolverbindungen (D), starke synergistische Verdickungseffekte erzielt werden.

## Tabelle 1<sup>a)</sup>

|  | V1 | V2 | V3 | V4 | V5 | V6 | V7 | V8 | V9 | V10 | B1 |
|---|---|---|---|---|---|---|---|---|---|---|---|
| Texapon SB3 | 4,8 | 4,8 | 4,8 | 4,8 | 4,8 | 4,8 | 4,8 | 4,8 | 4,8 | 4,8 | 4,8 |
| CT-12 | 7,2 | 7,2 | 7,2 | 7,2 | 7,2 | 7,2 | 7,2 | 7,2 | 7,2 | 7,2 | 7,2 |
| APG 600 | - | - | - | 1 | 3 | 5 | - | - | 1 | 3 | 3 |
| NaCl | 1 | 3 | 5 | - | - | - | - | - | 1 | 3 | 5 |
| GT-282 | - | - | - | - | - | - | 1 | 2 | - | - | 3 |
| Wasser | ad 100 | ad 100 | ad 100 | ad 100 | ad 100 | ad 100 | ad 100 | ad 100 | ad 100 | ad 100 | ad 100 |
| Viskosität [mPas] | < 50 | < 50 | < 50 | < 50 | < 50 | < 50 | < 50 | 100 | < 50 | < 50 | 1000 |

a) Die Angaben im oberen Block der Tabelle beziehen sich auf Gew.-% Aktivsubstanz bezogen auf die gesamte Mischung.

EP 0 592 475 B1

## Tabelle 2<sup>a)</sup>

| | V11 | V12 | V13 | V14 | V15 | V16 | V17 | V18 | V19 | B2 | B3 | B4 | B5 | B6 | B7 |
|---|---|---|---|---|---|---|---|---|---|---|---|---|---|---|---|
| Texapon N25 | 7,0 | 7,0 | 7,0 | 7,0 | 7,0 | 7,0 | 7,0 | 7,0 | 7,0 | 7,0 | 7,0 | 7,0 | 7,0 | 7,0 | 7,0 |
| Ölsäuresulfonat | 3,0 | 3,0 | 3,0 | 3,0 | 3,0 | 3,0 | 3,0 | 3,0 | 3,0 | 3,0 | 3,0 | 3,0 | 3,0 | 3,0 | 3,0 |
| APG 600 | - | - | - | 1,0 | 3,0 | 5,0 | - | - | - | 1,0 | 1,0 | 1,0 | 2,0 | 2,0 | 2,0 |
| NaCl | 1,0 | 3,0 | 5,0 | - | - | - | - | - | - | 1,0 | 1,0 | 1,0 | 3,0 | 3,0 | 3,0 |
| DOE-120 | - | - | - | - | - | - | 1,0 | - | - | 1,0 | - | - | 3,0 | - | 5,0 |
| Antil-141 | - | - | - | - | - | - | - | 1,0 | - | - | 1,0 | - | - | - | - |
| GT-282 | - | - | - | - | - | - | - | - | 1,0 | - | - | 1,0 | - | 3,0 | - |
| Wasser | b) | b) | b) | b) | b) | b) | b) | b) | b) | b) | b) | b) | b) | b) | b) |
| Viskosität [mPas] | <50 | <50 | <50 | <50 | <50 | <50 | <50 | <50 | <50 | 150 | 100 | 200 | 800 | 1000 | 2500 |

a) wie bei Tabelle 1

b) Wasser jeweils ad 100

EP 0 592 475 B1

Tabelle 3a)

|  | V20 | V21 | V22 | V23 | V24 | B8 | B9 |
|---|---|---|---|---|---|---|---|
| Texapon ASV | 6,0 | 6,0 | 6,0 | 6,0 | 6,0 | 6,0 | 6,0 |
| HMS | 4,0 | 4,0 | 4,0 | 4,0 | 4,0 | 4,0 | 4,0 |
| APG 600 | - | - | 2,0 | 2,0 | - | 1,0 | 1,0 |
| NaCl | 1,0 | 2,0 | - | 2,0 | - | 1,0 | 1,0 |
| DOE-120 | - | - | - | - | 1,0 | 1,0 | 2,0 |
| Wasser | ad 100 | ad 100 | ad 100 | ad 100 | ad 100 | ad 100 | ad 100 |
| Viskosität [mPas] | < 50 | < 50 | < 50 | 150 | 100 | 500 | 2000 |

a) wie bei Tabelle 1

Tabelle 4a)

|  | V25 | V26 | V27 | V28 | B10 |
|---|---|---|---|---|---|
| $C_{12/14}$-Disalz | 8,0 | 8,0 | 8,0 | 8,0 | 8,0 |
| Texapon ASV | 2,0 | 2,0 | 2,0 | 2,0 | 2,0 |
| APG 600 | - | - | 1,0 | - | 1,0 |
| NaCl | 1,0 | 2,0 | 1,0 | - | 1,0 |
| DOE-120 | - | - | - | 1,0 | 1,0 |
| Wasser | ad 100 | ad 100 | ad 100 | ad 100 | ad 100 |
| Viskosität [mPas] | 3200 | 2400 | 3300 | 3600 | 4200 |

a) wie bei Tabelle 1

**Patentansprüche**

1. Wäßrige Zubereitungen oberflächenaktiver Substanzen enthaltend

(A) 1 bis 30 Gew.-% eines oder mehrerer Aniontenside, die 1 oder 2 lipophile Reste mit 1 bis 22 C-Atomen und 1 bis 3 polare Reste ausgewählt aus der Gruppe der Carboxylat-, Sulfat- oder Sulfonatreste und gegebenenfalls einen Polyoxyalkylenrest mit einem mittleren Alkoxylierungsgrad von 1 bis 15 enthalten,

(B) ein oder mehrere Alkylglycoside der allgemeinen Formel (I), worin

$$RO\text{-}(G)_x \qquad (I)$$

R einen linearen, gesättigten Alkylrest mit 8 bis 22 C-Atomen und $(G)_x$ ein Glycosid- oder Oligoglycosidrest mit einem Oligomerisationsgrad x von 1 bis 4 ist,

(C) ein anorganisches Elektrolytsalz,

(D) ein oder mehrere Polyetherverbindungen, die 2 oder 3 lipophile Reste mit 8 bis 22 C-Atomen und 1 bis 3 Polyoxyalkylenreste mit 20 bis 250 Oxyalkylen-Einheiten enthalten,

wobei die Summe der Komponenten (B), (C) und (D) 0,5 bis 20 Gew.-% beträgt und nicht größer als der Gehalt an Komponente (A) ist, mit der Maßgabe, daß das Gewichtsverhältnis von Alkylglycosid (B) zu Elektrolyt (C) 5 : 1 bis 1 : 5 und das Gewichtsverhältnis von Alkylglycosid (B) zur Polyetherverbindung (D) 5 : 1 bis 1 : 3 beträgt.

2. Wäßrige Zubereitungen nach Anspruch 1, worin der Anteil der oberflächenaktiven Substanzen (A) 5 bis 25 Gew.-% beträgt.

3. Wäßrige Zubereitungen nach Anspruch 1 oder 2, worin die Summe der Komponenten (B), (C) und (D) 1,0 bis 10,0 Gew.-% beträgt.

**4.** Wäßrige Zubereitungen nach einem der Ansprüche 1 bis 4, worin die oberflächenaktiven Substanzen (A) ausgewählt werden aus der Gruppe der Alkylethersulfate, Dialkylethersulfate oder Sulfosuccinate.

**5.** Verwendung der Zubereitungen nach einem der Ansprüche 1 bis 5 als Reinigungsmittel, insbesondere zur Körperreinigung.

**Claims**

**1.** Water-containing preparations of surface-active substances containing

(A) 1 to 30% by weight of one or more anionic surfactants containing 1 or 2 lipophilic $C_{1-22}$ groups and 1 to 3 polar groups selected from carboxylate, sulfate or sulfonate groups and, optionally, a polyoxyalkylene group having an average degree of alkoxylation of 1 to 15,

(B) one or more alkyl glycosides corresponding to general formula (I):

$RO(G)_x$     (I)

in which R is a linear, saturated $C_{8-22}$ alkyl radical and $(G)_x$ is a glycoside or oligoglycoside unit having a degree of oligomerization x of 1 to 4,

(C) an inorganic electrolyte salt,

(D) one or more polyether compounds containing 2 or 3 lipophilic $C_{8-22}$ groups and 1 to 3 polyoxyalkylene groups bearing 20 to 250 oxyalkylene units,

the sum of components (B), (C) and (D) amounting to 0.5 to 20% by weight and being no greater than the content of component (A), with the proviso that the ratio by weight of alkyl glycoside (B) to electrolyte (C) is 5:1 to 1:5 and the ratio by weight of alkyl glycoside (B) to the polyether compound (D) is 5:1 to 1:3.

**2.** Water-containing preparations as claimed in claim 1, in which the percentage content of surfactants (A) is 5 to 25% by weight.

**3.** Water-containing preparations as claimed in claim 1 or 2, in which the sum of components (B), (C) and (D) is 1.0 to 10.0% by weight.

**4.** Water-containing preparations as claimed in any of claims 1 to 3, in which the surfactants (A) are selected from the group of alkyl ether sulfates, dialkyl ether sulfates or sulfosuccinates.

**5.** The use of the preparations claimed in any of claims 1 to 4 as cleaning compositions, more particularly for personal hygiene.

**Revendications**

**1.** Préparations aqueuses de substances tensioactives contenant:

(A) 1 à 30 % en poids d'un ou de plusieurs tensioactifs anioniques qui renferment 1 ou 2 radicaux lipophiles ayant 1 à 22 atomes de C et 1 à 3 radicaux polaires choisis dans le groupe des radicaux carboxylate, sulfate ou sulfonate et éventuellement un radical polyoxyalkylène avec un degré d'alcoxylation moyen de 1 à 15.

(B) un ou plusieurs alkylglycosides de formule générale (I)

$RO(G)_x$     (I)

dans laquelle,

R est un alkyle linéaire saturé avec 8 à 22 atomes de C et $(G)_x$ un radical glycoside ou oligoglycoside avec un degré d'oligomérisation x de 1 à 4.

(C) un sel minéral d'électrolyte,

(D) un ou plusieurs composés de polyéthers, qui contiennent 2 à 3 radicaux lipophiles avec 8 à 22 atomes de C et 1 à 3 radicaux polyoxyéthylène avec 20 à 250 unités oxyalkylène,

la somme des composants (B), (C) et (D) se monte à 0,5 jusqu'à 20 % en poids et n'est pas supérieure à la teneur en composant (A),

sous réserve que le rapport de poids d'alkylglucoside (B) à l'électrolyte (C) se monte de 5:1 à 1:5 et que le rapport de poids d'alkylglycoside (B) au composé polyéther (D) soit compris de 5:1 à 1:3.

2.  Préparations aqueuses selon la revendication 1, dans laquelle la proportion de substances tensioactives (A) se situe de 5 à 25 % en poids.

3.  Préparations aqueuses selon la revendication 1 ou 2, dans laquelle la somme des composants (B), (C) et (D) va de 1,0 à 10,0.

4.  Préparations aqueuses selon une des revendications 1 à 4, dans laquelle les substances tensioactives (A) sont choisies dans le groupe des éthers sulfates d'alkyle, des éthers sulfates de dialkyle ou des sulfosuccinate.

5.  Utilisation des préparations selon une des revendications 1 à 5 en tant que produit de lavage, en particulier pour le propreté corporelle.